(19) Europäisches Patentamt
European Patent Office
Office européen des brevets

(11) Numéro de publication : **0 370 902 B1**

# (12) FASCICULE DE BREVET EUROPEEN

(45) Date de publication du fascicule du brevet : **26.08.92 Bulletin 92/35**

(51) Int. Cl.$^5$ : **A61K 31/40,** A61K 31/44, A61K 31/50

(21) Numéro de dépôt : **89403221.8**

(22) Date de dépôt : **22.11.89**

## (54) Utilisation de dérivés du chromanne pour preparer un medicament utile pour le traitement des états dépressifs.

(30) Priorité : **23.11.88 FR 8815283**

(43) Date de publication de la demande : **30.05.90 Bulletin 90/22**

(45) Mention de la délivrance du brevet : **26.08.92 Bulletin 92/35**

(84) Etats contractants désignés : **AT BE CH DE ES FR GB GR IT LI LU NL SE**

(56) Documents cités :
**EP-A- 0 115 142**
**EP-A- 0 273 262**
**NAUNYN-SCHMIEDEBERG'S ARCH. PHARMACOL., vol. 337, no. 4, avril 1988, pages 429-434, Springer-Verlag; C. ALZHEIMER et al.:"Actions of BRL 34915 (Cromakalim) upon convulsive discharges in guinea pig hippocampal slices"**

(56) Documents cités :
**INDIAN JOURNAL OF CHEMISTRY, vol. 21B, avril 1982, pages 344-347; R.C. GUPTA et al.: "Chromene & chroman 3-carboxamides& some related compounds as a new class of centrally acting agents"**

(73) Titulaire : **ELF SANOFI**
**32-34, rue Marbeuf**
**F-75008 Paris (FR)**

(72) Inventeur : **Soubrie, Philippe**
**Le Rey Valflaunès**
**F-34270 Saint Mathieu de Treviers (FR)**
Inventeur : **Poncelet, Martine**
**8, rue des Fraisiers**
**F-34000 Montpellier (FR)**

(74) Mandataire : **Gillard, Marie-Louise et al**
**Cabinet Beau de Loménie 55, Rue d'Amsterdam**
**F-75008 Paris (FR)**

EP 0 370 902 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne l'utilisation de dérivés du chromanne pour la préparation de compositions pharmaceutiques destinées à combattre la dépression, notamment utilisables dans une méthode pour le traitement des états dépressifs, ainsi que des compositions pharmaceutiques pour le traitement de la dépression.

Depuis quelques années, les demandes de brevets européens publiées sous les n° 76 075, 91 748, 93 535, 120 426 et 273 262 ont décrit des familles de dérivés du chromannol-3 et du 2H-chromène présentant en commun une activité antihypertensive.

La présente invention a pour objet l'utilisation de dérivés du chromannol ou du chromène pour le traitement des états dépressifs.

Selon la présente invention, les composés de formule (I) ci-dessous ainsi que leurs sels pharmaceutiquement acceptables peuvent être utilisés pour le traitement de la dépression

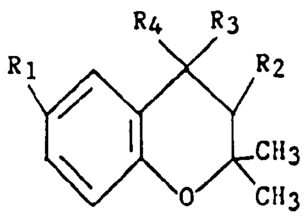

(I)

Dans cette formule,

– $R_1$ représente l'hydrogène, un atome d'halogène, un groupe cyano, un groupe nitro, un groupe acétyle, un groupe trifluorométhyle ;

– $R_2$ représente un groupe hydroxyle et, dans ce cas, $R_3$ représente un atome d'hydrogène ou encore $R_2$ et $R_3$ pris ensemble représentent une liaison supplémentaire entre les atomes de carbone qui les portent ;

– $R_4$ représente un groupe oxo-2 pyrrolidinyl-1 ou oxo-2 pipéridyl-1 ou encore un groupe hétérocyclique choisi parmi les groupes 1H-2-pyridon-1-yl, 1H-6-pyridazinon-1-yl, 1H-2-pyrimidinon-1-yl, 1H-6-pyrimidinon-1-yl, 1H-2-pyrazinon-1-yl ou 1H-2-thiopyridon-1-yl non substitués ou éventuellement substitués 1 ou 2 fois par un groupe alkyle en $C_1$-$C_4$, un atome d'halogène, un groupe hydroxyle, nitro, amino, carboxyle.

Des exemples de tels composés figurent dans les demandes de brevets européens publiées citées précédemment.

Les composés de formule (I) où $R_2$ est un hydroxyle sont asymétriques et, par suite, peuvent exister sous forme d'isomères optiques. La présente invention concerne aussi bien les isomères individuellement que leurs mélanges ou les composés racémiques.

Les composés de formule (I) peuvent être préparés comme décrit dans les demandes de brevets européens citées précédemment ou par des procédés analogues.

L'administration des composés de formule (I) peut être effectuée par voie orale, sublinguale, transdermique ou par voie parentérale.

La quantité de principe actif à administrer pour traiter les états dépressifs dépend, comme il est habituel, de la nature et de la sévérité des affections à traiter ainsi que du poids des malades.

Toutefois, les unités de dosage contiendront habituellement de 5 à 500 mg d'ingrédient actif en combinaison avec un support pharmaceutique.

Cette dose unitaire peut être administrée de 1 à 5 fois par jour pour conduire à un dosage journalier de 25 à 2 500 mg et de préférence de 50 à 1 500 mg.

Dans les compositions pharmaceutiques de la présente invention pour l'administration orale, sublinguale, sous-cutanée, intramusculaire, intraveineuse ou rectale, les principes actifs de formule (I) ci-dessus, ou leurs sels éventuels, peuvent être administrés sous formes unitaires d'administration, en mélange avec des supports pharmaceutiques classiques, aux êtres humains pour la prophylaxie ou le traitement des troubles ou des maladies ci-dessus. Les formes unitaires d'administration appropriées comprennent les formes par voie orale, telles que les comprimés, les gélules, les poudres, les granules et les solutions ou suspensions orales, les formes d'administration sublinguale, les formes d'administration sous-cutanée, intramusculaire ou intraveineuse et les formes d'administration rectale.

Lorsqu'on prépare une composition solide sous forme de comprimés, on mélange l'ingrédient actif principal avec un véhicule pharmaceutique, tel que la gélatine, l'amidon, le lactose, le stéarate de magnésium, le talc, la gomme arabique ou analogues. On peut enrober les comprimés de saccharose, d'un dérivé cellulosique, ou d'autres matières appropriées ou encore on peut les traiter de telle sorte qu'ils aient une activité prolongée

ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir ou pour l'administration sous forme de gouttes peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents de mise en suspension, comme la polyvinylpyrrolidone, de même qu'avec des édulcorants ou des correcteurs du goût

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des agents mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Les résultats pharmacologiques rapportés ci-après illustrent l'activité de composés de formule (I) dans un test indicatif de composés d'usage potentiel dans le traitement des états dépressifs.

Les composés testés sont les suivants :

Composé 1 : Trans cyano-6 diméthyl-2,2 (oxo-2 pyrrolidinyl-1)-4 chromanne-3-ol
Composé 2 : Trans cyano-6 diméthyl-2,2 (1H—oxo-2 pyridyl-1)-4 chromanne-3-ol
Composé 3 : Trans cyano-6 diméthyl-2,2 (chloro-4 1H-oxo-2 pyridyl-1)-4 chromanne-3-ol
Composé 4 : Trans cyano-6 diméthyl-2,2 (1H-oxo-6 pyridazinyl-1)-4 chromanne-3-ol
Composé 5 : Trans cyano-6 diméthyl-2,2 (hydroxy-3 1H-oxo-6 pyridazinyl-1)-4 chromanne-3-ol
Composé 6 : Trans diméthyl-2,2 (1H-oxo-2 pyridyl-1)-4 chromanne-3-ol
Composé 7 : Cyano-6 diméthyl-2,2 (1H-oxo-2 pyridyl-1)-4 2H-chromène
Composé 8 : Acétyl-6 diméthyl-2,2 (1H-oxo-2 pyridyl-1)-4 2H-chromène
Composé 9 : Bromo-6 diméthyl-2,2 (1H-oxo-2 pyridyl-1)-4 2H-chromène
Composé 10 : Amino-6 diméthyl-2,2 (1H-oxo-2 pyridyl-1)-4 2H-chromène
Composé 11 : Trans cyano-6 diméthyl-2,2 (1H-oxo-2pyridyl-1)-4-chromanne-3-ol, isomère lévogyre
Composé 12 : Bromo-6 diméthyl-2,2 dihydro-1,2 oxo-2 pyridyl-1)-4 chromène

Le composé 11 a été préparé à partir du composé racémique correspondant, le composé 2, en utilisant une méthode connue, qui consiste à préparer de façon intermédiaire un ester carbamique optiquement actif selon le procédé décrit dans la demande de brevet européen 120 428 :

Préparation du composé 11 :

On maintient à reflux pendant 24 h un mélange contenant 6,5 g du composé 2, 180 ml de toluène et 6 g de S(-)isocyanate d'α-méthylbenzyle. On concentre sous vide et on reprend le résidu par 300 ml d'éther éthylique. L'insoluble est filtré puis cristallisé dans 160 ml d'acétate d'éthyle. On obtient 1 g de produit puis on recristallise 2 fois dans un mélange acétate d'éthyle-pentane (1/1). On obtient 0,8 g du carbamate d'α-méthylbenzyle.

Point de fusion : 280°C.

$\alpha_D(23)$ : -8,5 (acétone C = 1).

On laisse sous agitation pendant 24 h un mélange contenant 0,7 g du composé obtenu précédemment, 3 ml de chloroforme, 1,5 ml de triéthylamine et 1,4 ml de trichlorosilane à 95 %. On verse sur de la glace puis on extrait au chloroforme, filtre l'insoluble et traite par du méthanol chaud. La phase organique est concentrée sous vide pour obtenir 0,9 g d'un solide que l'on chromatographie sur colonne de silice en éluant par un mélange dichlorométhane-acétate d'éthyle (7/3). On obtient 0,4 g du produit attendu.

Point de fusion : 260-265°C.

$\alpha_D(23)$ : -43,0 (chloroforme, C = 1).

L'analyse des spectres IR et RMN confirme la structure.

Test de Porsolt

Les produits selon l'invention ont été soumis au test de la nage forcée selon Porsolt et al. (Archives Internationales de Pharmacodynamie, 1977, 229, 327-336).

Des souris femelles (Iffa Credo) de 22 à 24 g sont plongées pour une durée de 6 min dans un bécher contenant 800 ml d'eau à 24 ± 1°C.

En l'absence de traitement, après une brève période d'activité, les animaux adoptent une posture caractéristique immobile. L'immobilité est réduite par des substances présentant une activité antidépressive, alors que les tranquillisants sont sans effet.

Les produits à étudier sont administrés par voie intrapéritonéale à raison de 10 animaux par dose et les animaux sont soumis au test 30 min plus tard. Les temps d'immobilité sont mesurés pendant 4 min de la 2e à la 6e minute d'expérience.

Les résultats sont exprimés en pourcentage de variation du temps d'immobilité par rapport à un lot d'animaux témoins non traités.

Les résultats obtenus sont rassemblés dans le tableau suivant :

| Produit | Dose mg/kg Voie i.p. | % Variation du temps d'immobilité |
|---|---|---|
| Composé 1 | 0,25 | -26** |
| Composé 2 | 0,5 | -24** |
| | 2 | -30*** |
| Composé 3 | 0,06 | -25* |
| Composé 4 | 0,25 | -31* |
| Composé 5 | 0,5 | -27* |
| Composé 6 | 1 | -13* |
| Composé 7 | 0,125 | -21*** |
| | 0,25 | -32*** |
| | 0,5 | -44*** |
| Composé 8 | 0,25 | -28** |
| Composé 9 | 0,03 | -22* |
| | 0,125 | -35** |
| Composé 10 | 1 | -36** |
| Composé 11 | 1 | -40** |
| Composé 12 | 0,125 | -35* |

Test t de Student : * $p < 0{,}05$, ** $p < 0{,}01$, *** $p < 0{,}001$

## Revendications

1. Utilisation d'un composé de formule :

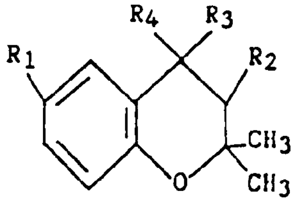

(I)

dans laquelle :

– $R_1$ représente l'hydrogène, un atome d'halogène, un groupe nitro, un groupe acétyle, un groupe trifluorométhyle , un groupe cyano ;

– $R_2$ représente un groupe hydroxyle et, dans ce cas, $R_3$ représente un atome d'hydrogène ou encore $R_2$ et $R_3$ pris ensemble représentent une liaison supplémentaire entre les atomes de carbone qui les portent ;

– $R_4$ représente un groupe oxo-2 pyrrolidinyl-1 ou oxo-2 pipéridyl-1 ou encore un groupe hétérocyclique choisi parmi les groupes 1H-2-pyridon-1-yl, 1H-6-pyridazinon-1-yl, 1H-2-pyrimidinon-1-yl, 1H-6-pyrimidinon-1-yl, 1H-2-pyrazinon-1-yl ou 1H-2-thiopyridon-1-yl non substitués ou éventuellement substitués 1 ou 2 fois par un groupe alkyle en $C_1$-$C_4$, un atome d'halogène, un groupe hydroxyle, nitro, amino, carboxyle ou d'un de leurs sels pharmaceutiquement acceptables pour préparer un médicament utile pour le traitement des états dépressifs.

2. Utilisation selon la revendication 1, dans laquelle $R_2$ représente un hydroxyle et $R_3$ est l'hydrogène.

3. Utilisation selon la revendication 1, dans laquelle $R_2$ et $R_3$ pris ensemble constituent une liaison.

4. Utilisation selon la revendication 1, dans laquelle le composé de formule (I) est choisi parmi :

le trans cyano-6 diméthyl-2,2 hydroxy-3 (chloro-4 1H-oxo-2 pyridyl-1)-4 chromanne,
le cyano-6 diméthyl-2,2 (1H-oxo-2 pyridyl-1)-4 2H-chromène.

## Patentansprüche

1. Verwendung einer Verbindung der Formel

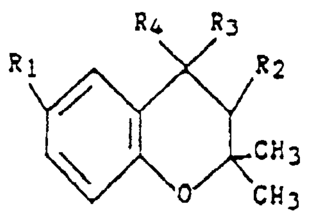

(I)

worin:

– $R_1$ Wasserstoff, ein Halogenatom, eine Nitrogruppe, eine Acetylgruppe, eine Trifluormethylgruppe oder eine Cyanogruppe darstellt;

– $R_2$ eine Hydroxylgruppe darstellt, in welchem Fall $R_3$ ein Wasserstoffatom ist, oder aber $R_2$ und $R_3$, zusammen genommen, eine zusätzliche Bindung zwischen den Kohlenstoffatomen, die sie tragen, bilden;

– $R_4$ eine 2-Oxopyrrolidin-1-yl- oder 2-Oxopiperid-1-yl-gruppe oder aber eine nicht substituierte oder gegebenenfalls ein- oder zweimal durch eine $C_1$-$C_4$-Alkylgruppe, ein Halogenatom, eine Hydroxyl-, Nitro-, Amino- oder Carboxylgruppe substituierte heterocyclische Gruppe, ausgewählt aus den Gruppen 1H-2-Pyridon-1-yl, 1H-6-Pyridazinon-1-yl, 1H-2-Pyrimidinon-1-yl, 1H-6-Pyrimidinon-1-yl, 1H-2-Pyrazinon-1-yl oder 1H-2-Thiopyridon-1-yl, darstellt;

oder eines ihrer pharmazeutisch akzeptablen Salze zur Herstellung eines zur Behandlung von depressiven Zuständen geeigneten Medikaments.

2. Verwendung nach Anspruch 1, worin $R_2$ ein Hydroxyl darstellt und $R_3$ Wasserstoff ist.

3. Verwendung nach Anspruch 1, worin $R_2$ und $R_3$, zusammen genommen, eine Bindung bilden.

4. Verwendung nach Anspruch 1, worin die Verbindung der Formel (1) ausgewählt ist aus:

trans-6-Cyano-2,2-dimethyl-3-hydroxy-4-(4-chlor-1H-2-oxo-pyrid-1-yl)-chroman und
6-Cyano-2,2-dimethyl-4-(1H-2-oxo-pyrid-1-yl)-2H-chromen.

## Claims

1. Use of a compound of the formula

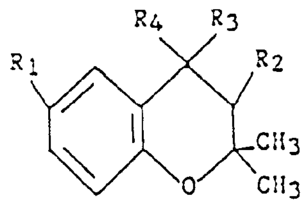

(I)

in which:

- $R_1$ represents hydrogen, a halogen atom, a nitro group, an acetyl group, a trifluoromethyl group ; a cyano group ;
- $R_2$ represents a hydroxyl group, in which case $R_3$ represents a hydrogen atom, or else $R_2$ and $R_3$ taken together represent an additional bond between the carbon atoms by which they are carried;
- $R_4$ represents a 2-oxopyrrolidin-1-yl or 2-oxopiperid-1-yl group or else a heterocyclic group selected from 1H-pyrid-2-on-1-yl, 1H-pyridazin-6-on-1-yl, 1H- pyrimidin- 2-on-1-yl, 1H-pyrimidin-6-on-1-yl, 1H-pyrazin-2-on-1-yl and 1H-thiopyrid-2-on-1-yl groups which are unsubstituted or optionally monosubstituted or disubstituted by a $C_1$-$C_4$ alkyl group, a halogen atom or a hydroxyl, nitro, amino or carboxyl group or one of the pharmaceutically acceptable salts thereof, for the preparation of a drug for the treatment of depressive states.

2. Use according to claim 1, wherein $R_2$ represents a hydroxyl and $R_3$ is hydrogen.

3. Use according to claim 1, wherein $R_2$ and $R_3$ taken together form a bond.

4. Use according to claim 1, wherein the compound of formula (I) is selected from: trans-6-cyano-2,2-dimethyl-3-hydroxy-4-(4-chloro-1H-2-oxopyrid-1-yl)chroman and 6-cyano-2,2-dimethyl-4-(1H-2-oxopyrid-1-yl)-2H-chromene.